Europäisches Patentamt

European Patent Office

Office européen des brevets

(11) Publication number: **0 300 552**
**A1**

## EUROPEAN PATENT APPLICATION

(21) Application number: **88201471.5**

(51) Int. Cl.⁴: **A61M 5/14**

(22) Date of filing: **12.07.88**

(30) Priority: **13.07.87 NL 8701644**

(43) Date of publication of application:
**25.01.89 Bulletin 89/04**

(84) Designated Contracting States:
**DE FR GB IT NL**

(71) Applicant: **CORDIS EUROPA N.V.**
**Oosteinde 8**
**NL-9301 LJ Roden(NL)**

(72) Inventor: **Meinema, Ate Jelle**
**Schonauwen 35**
**NL-9301 SN Roden(NL)**

(74) Representative: **Smulders, Theodorus A.H.J.**
**et al**
**Vereenigde Octrooibureaux Nieuwe Parklaan**
**107**
**NL-2587 BP 's-Gravenhage(NL)**

(54) An infusion system for infusion of fluids into a living body.

(57) An infusion system is provided for purposes of infusing fluids, such as insulin, into a living body. A housing (2) is implanted within the living body and contains a reservoir of fluid, such as insulin, and an actuatable pump (6) for, when actuated, pumping a quantity of the fluid for delivery into the body as with a catheter (9) extending from the pump (6) to the body site. A pump actuation controller (110), which is normally electrically passive, is mounted within the housing for purposes of actuating the pump. The controller includes an antenna (12) for receiving an RF signal through the body from an external transmitter (4). The RF signal is converted into a DC signal for charging a capacitor (14). A voltage comparator (15) compares the charge level of the capacitor (14) with a reference level and provides a trigger signal when the levels attain a predetermined relationship. A capacitor discharge circuit is provided which includes a first actuatable switch (16), actuated by the trigger signal, and the pump. The capacitor is discharged when the switch is actuated for purposes of actuating the pump to thereby pump a quantity of the fluid for delivery into the body. This is accomplished only in response to the receiving antenna (12) having received an RF signal.

FIG.1

## An infusion system for infusion of fluids into a living body

This invention relates to the art of implantable infusion devices for infusing fluids, such as insulin, into a living body.

More particularly this invention relates to the art of implantable devices for locally administering a liquid analgesic such as morphine in a patient's living body.

Implantable infusion devices are known in the art for delivering fluid to a living body. For example, the U.S. Patent to Prestele et al. 4,395,259 discloses an implantable infusion device for delivering medication, into the human body. Prestele includes an external transmitter for communicating coded signals to a receiver located within an implanted housing which includes a delivery pump for deliverying fluid to the body. The implanted receivng circuitry for controlling the pump includes a battery for providing the electrical power required. It is apparent that when the battery is no longer active, the battery must be surgically removed and a new battery be surgically inserted.

Another implantable infusion system in the prior art takes the form of that disclosed in the U.S. Patent to Portner 4,360,019. Portner discloses a system wherein the implanted device includes an electronic control circuit for controlling a pump together with a battery for providing electrical power to the electronic control circuit.

Portner also discloses a receiving coil for receiving an RF signal, from an external transmitter, together with means for rectifying the received signal to provide a DC signal for charching a pulse capacitor. The battery supplied electronic control circuit provides the trigger signal for closing a switch for discharging the capacitor through a solenoid to activate the pump. Thus, it is seen that internal battery power is required for operating the internal circuitry in order to trigger the switch for discharging the capacitor.

Another approach taken in the prior art is illustrated in the U.S. Patent to Fischell 4,373,527. Fischell employs a rechargeable power cell which is contained within an implanted housing for providing the necessary electrical power for operating the circuitry to activate a pump. Additionally, Fischell discloses the use of a pick-up coil mounted in the implantable housing for receiving an AC signal from an external transmitter. The AC signal is then rectified and supplied to a battery charge control circuit which maintains the power cell at a charged level. Such chargeable cells may be dangerous and may explode, when shorted. Consequently, they must be handled carefully. Such cells should be avoided in applications wherein an implanted device requires electrical power for activating a pump for infusion of liquids into a living body. Moreover, Fischell's circuitry requires decoding circuitry for decoding signals which are telemetrically transmitted through the patient's skin.

It is a primary object of the present invention to provide an infusion system including an implantable housing which contains no battery or power cell, but instead incorporates normally electrically passive pump actuation control circuitry which is operative to activate the pump only when an implanted antenna receives an RF signal from an external transmitter.

It is a still further object of the present invention to provide such a system including an external transmitter having a position detector to find the correct positioning of the tranmitter relative to the implanted infusion device prior to transmitting RF energy of a sufficient level to actuate the implanted pump.

It is a still further object of the present invention to provide such a system wherein the transmitter receives information from the implanted electronic controller indicative that a pump actuation has taken place.

In accordance with the present invention, the infusion system includes a housing adapted to be implanted within a living body. The housing contains a reservoir of fluid, such as an analgesic, for example morphine, and an actuatable pump for, when actuated, pumping a quantity of the fluid for delivery into the body. A pump actuation controller is mounted within the housing and is normally electrically passive and serves to actuate the pump only when the antenna mounted within the housing receives an RF signal through the body from an external transmitter. The received RF signal is converted into a DC signal for charging a capacitor within the housing. The controller also includes a voltage comparator which compares the charge level of the capacitor with a reference level and provides a trigger signal when the levels attain a predetermined relationship. At that point, the capacitor is discharged through a circuit that includes a first actuatable switch, actuated by the trigger signal, and the actuatable pump so as to thereby actuate the pump to pump a quantity of the fluid for delivery into the body .

In accordance with a still further aspect of the present invention, the system is provided with a pump action verification circuit for verifying that a pump action has taken place. The circuit includes means within the implanted housing for short-circuiting the receiving antenna upon actuation of the pump so as to cause sudden change in the loading on the transmitter. The external transmitter is provided with circuitry for responding to this change in antenna loading as pump action verification. If a pump action verification is not obtained within a predetermined period of time, a failure indication will be presented to the operator and the transmitter will be deactivated.

In accordance with a still further aspect of the present invention, the system is provided with a position detector to detect when the transmitting antenna is properly positioned relative to the implanted receiving antenna before activating the pump. To this end, the transmitter has two modes of operation, a pulse mode for intermittently transmitting RF energy at a rate insufficient to charge the capaciter sufficient to activate the pump and a continuous mode for charging the capacitor. Initially, the transmitter is in its pulse mode and the transmitter includes circuitry for monitoring the loading of the transmitting antenna. As long as the transmitter is not in proper position, essentially no loading takes place. However, as the correct position is found, the loading will increase. This is detected by a position detector within the transmitter which then activates the transmitter to its continuous mode of operation so as to activate the implanted pump.

The foregoing and other objects and advantages of the invention will become more readily apparent from the following description of the preferred embodiment of the invention as taken in conjunction with the accompanying drawings which are a part hereof and wherein:

Fig. 1 is a schematic perspective view illustrating one embodiment of the invention for local administration of a liquid analgesic including an external transmitter and a receiver adapted to be implanted within a living body;

Fig. 2 is a block diagram illustration of the implanted control circuitry for operating the pump;

Fig. 3 is a schematic illustration of the control circuitry of Fig. 2;

Fig. 4 is a block diagram of the transmitter of Fig. 1;

Fig. 5 is a circuit diagram of the circuitry employed in the block diagram of Fig. 4; and

Fig. 6 is a block diagram of a programmable microprocessor based transmitter which may be employed in practicing the invention.

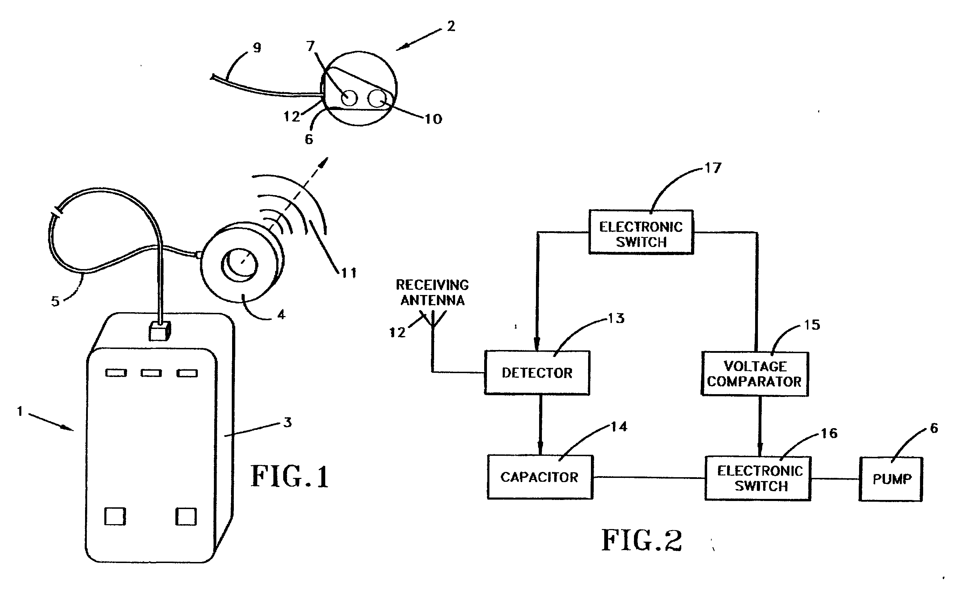

Reference is now made to the drawings wherein the showings are for purposes of illustrating preferred embodiments of the invention only and not for purposes of limiting the same. The infusion system is illustrated in Fig. 1 and includes an external control unit 1 located outside a patient's body and an implantable device, including a housing 2 to be implanted within a patient's body. The external control unit 1 includes a housing 3 containing the electronic circuitry, to be described in greater detail hereinafter, for controlling the transmission of RF energy by means of an antenna 4 connected to the electronic circuitry by means of a suitable conductor 5.

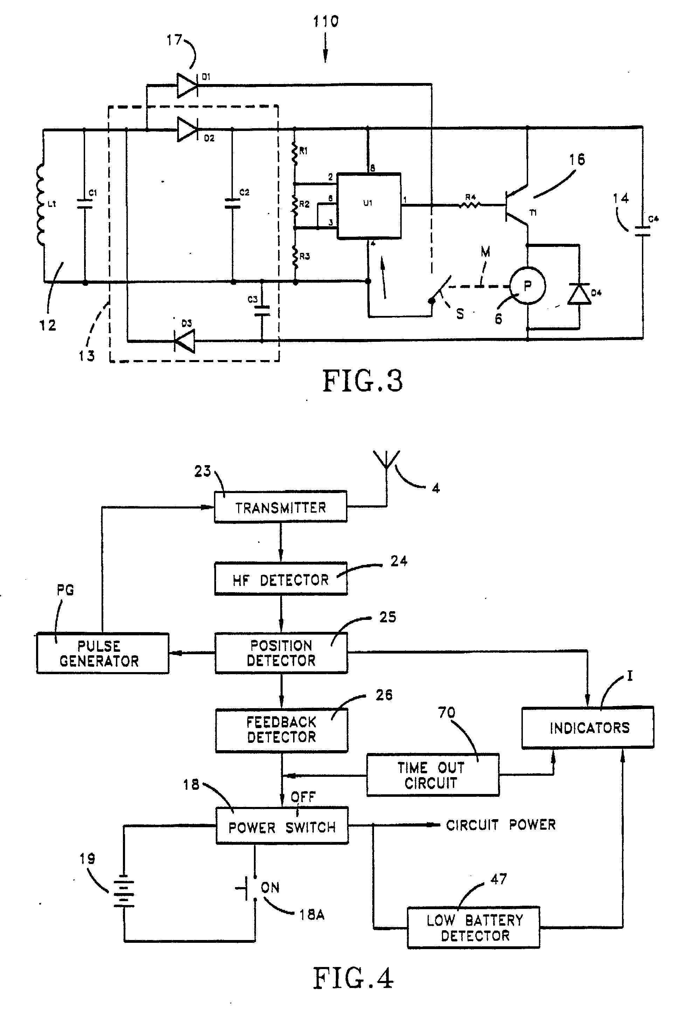

The implanted device 2 includes internal electronic circuitry (Figs. 2 and 3) for receiving the RF energy transmitted by the control unit 1 and converting the same to a form suitable for driving a pump. The housing contains a pump 6, a filling gate 7 for feeding liquid analgesic through the patient's skin, as with a hypodermic needle, to a liquid reservoir connected to the pump within the housing. The catheter 9 extends from the pump for delivering a quantity of pumped liquid to the location in the body where it is to be administered. The housing also includes a general access gate 10 for allowing access into the implant structure. A receiving antenna 12 is incorporated into the structure for receiving the RF energy 11 transmitted through the patient's skin from the external transmitting antenna 4. The details of the structure of the implanted device 2, other than the electronic controls, is not described in detail herein and suitable structure for this purpose may be found in the disclosure presented in the aforesaid U.S. Patent to Portner 4,360,019. The description which follows herein is specifically directed to the electronic control circuitry to which attention is now directed.

The implanted electronic control circuitry 110 includes the receiving antenna 12 which receives RF energy from the transmitting antenna and this RF energy is detected by a high frequency detector circuit 13 which incorporates a voltage doubler and rectifying circuit and which converts the RF signal into DC voltage for charging a capacitor 14. A voltage comparator 15 insures that upon a predetermined charge level, the capacitor 14 is discharged by way of a first electronic switch 16 across the pump 6 causing the pump to make one pump action so that a fixed quantity of liquid is discharged by way of the catheter 9 (Fig. 1) into the living body, simultaneously with the discharge of capacitor 14, the receiving antenna 12 is short-circuited by way of a second electronic switch 17 for a short time duration, on the order of 500 milliseconds. This is detected by the transmitter circuitry as an indication that the pump has been actuated. It is contemplated that the second electronic switch 17 may also be mechanically connected to the pumping mechanism so that the switch is closed to provide the short circuit operation in response to a pump action.

The circuitry illustrated in the block diagram of Fig. 2 may be implemented as shown by the circuit diagram of Fig. 3 to which attention is now directed. The receiving antenna 12 includes an inductor coil L1 which, together with a parallel capacitor C1, is tuned to 2 MHz. The high frequency detector 13 includes diodes D2, D3 and capacitors C2 and C3, connected as shown, and which serve to convert the received RF energy into a DC voltage with a voltage doubling action. This circuit, then, serves to charge the capacitor 14 (C4). The capacitor 14 charges up and the charge level is monitored by the comparator 15 which includes voltage dividing registers R1, R2, R3 connected as shown to an integrated circuit supplied by Intersil and known as their Model ICL7665BCJA. When the charge level across the capacitor reaches a predetermined level, as determined by the ratio of the resistors in the voltage dividing network, the voltage comparator will provide an output trigger signal which is supplied through resistor R4 to the base of a transistor 16 (T1) causing the transistor to conduct. Capacitor 14 will now discharge through the emitter-to-collector path of the transistor causing current to flow through a pump solenoid coil 6 (P). This, in turn, causes the pump to make one pump action whereby fluid from the implanted reservoir is pumped into the living body, as by catheter 9 (Fig. 1). When this pump action occurs, diode DI becomes forward biased so as to short-circuit the antenna 12 which generates a change in the load to the transmitter. This is detected within the transmitter, as will be described in greater detail hereinafter. A modification of this may be a mechanical interconnection M to a switch S from the pump 6 so that with each pump action the switch S is momentarily closed to complete a circuit for diode D1 so as to short-circuit the antenna. This modification is shown in dotted lines in Fig. 3.

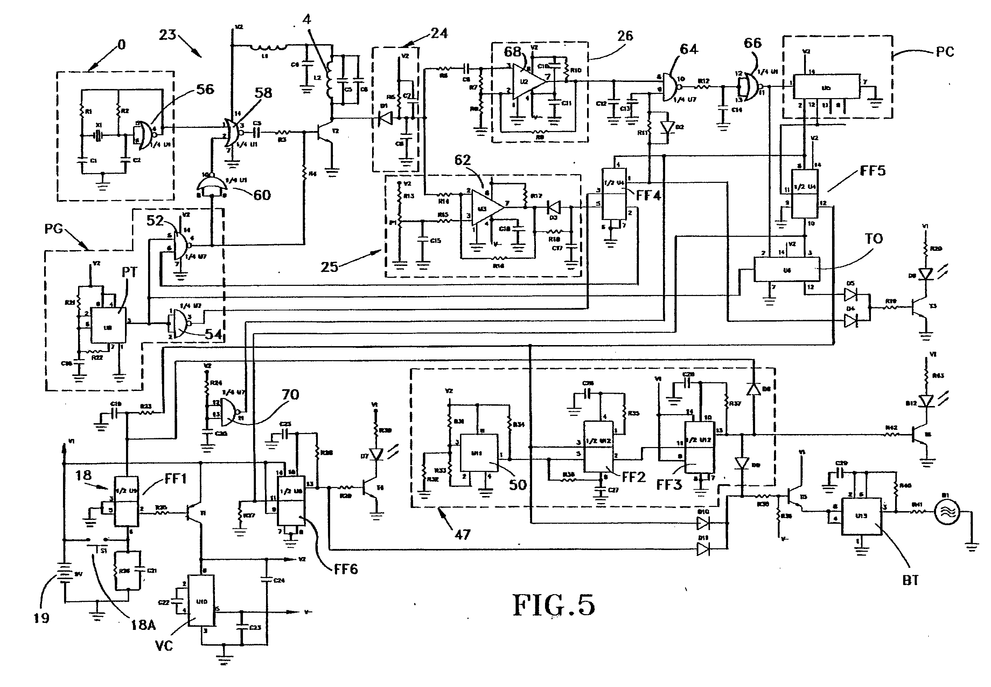

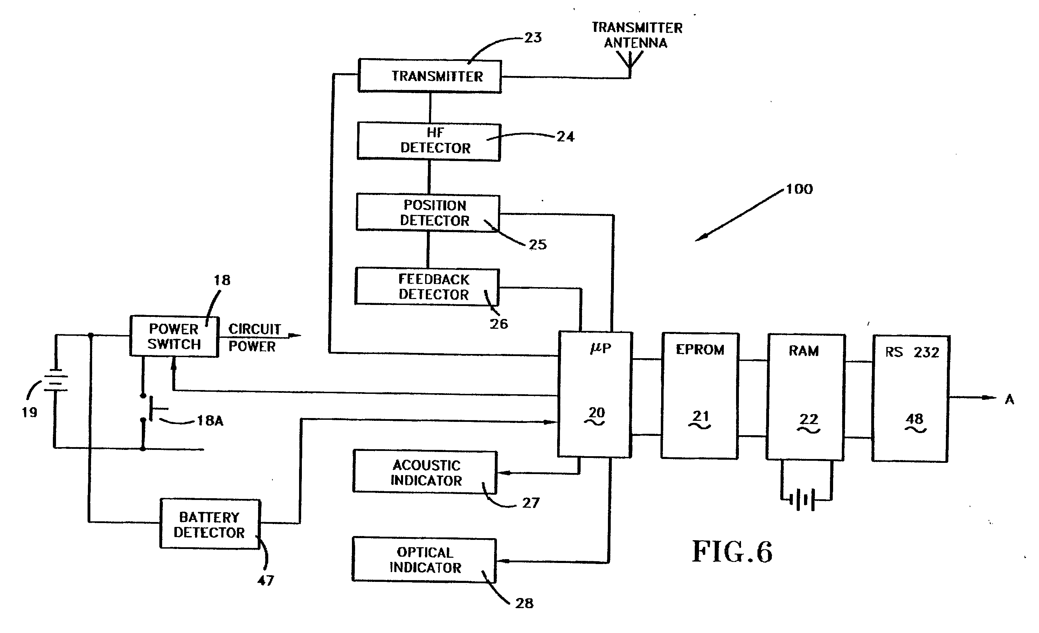

The pump 6 may take the form of a peristaltic pump, sometimes referred to as a finger pump or a roller pump. These pumps, known in the art, and are commercially available. Figs. 4, 5 and 6 illustrate the transmitter electronics mounted within housing 3 (Fig. 1). Fig. 4 presents a block diagram of one embodiment of the transmitter electronics with the circuitry hereof being illustrated in greater detail in Fig. 5. Fig. 6 illustrates a block diagram of a programmable microprocessor embodiment of the invention.

Attention is now directed to the embodiment of the invention as illustrated in the block diagram of Fig. 4 to be followed by a description thereof with respect to the circuitry illustrated in Fig. 5. In operation, the circuitry is activated by closing switch 18A which connects a 9 volt battery 19 with a power switch 18 to provide circuit power. A low battery detector 47 monitors the circuit power and activates an indicator I in the event of a low battery condition. The transmitter has two modes of operation, including a pulse mode and a continuous mode. Initially, after closure of switch 18A, the transmitter is in a pulse mode during which pulses are intermittently transmitted by antenna 4 which are not sufficient to cause the capacitor 14 (Fig. 3) to be charged to a level to cause actuation of the pump. During this pulse mode of operation, the pulse generator PG supplies pulses to gate the transmitter 23 for transmission by way of the antenna 4. The high frequency detector 24 detects these pulses as the antenna 4 is being moved about the exterior of the patient's body seeking a position at which energy will be most effective. The load on the antenna is monitored by the position detector 25. As long as the antenna is not in its proper position, there will be essentially no load noted by the position detector. Once the correct position is found, the transmitter load will increase sufficiently to cause the detector to switch the pulse generator PG to operate at a continuous mode. Additionally, the position detector will activate the indicator I to activate a ready indicator which then changes from a flashing mode to a continuous state.

It is during this continuous state that the transmitter transmits a continuous RF signal sufficient for the capacitor 14 (Fig. 3) to charge to a level to actuate the pump 6 to provide one pump action. A feedback detector 26 monitors the operation during the continuous mode to determine whether the pump action has taken place. Whenever such a pump action does take place, there is a sudden change in the transmitter load which appears as a short step in the detected high frequency signal detected by detector 24. This is detected by the feedback detector to activate the indicator I to signify that a pump action has taken place. In the event that a pump action does not take place within a predetermined time after switch 18A has been closed, a time out circuit TO will respond to this condition and activate a failure indicator and turn off the power switch 18 to remove the circuit power. The means by which these functions and others are attained is discussed in greater detail hereinafter with reference to the circuit diagram of Fig. 5.

Reference is now made to Fig. 5 wherein like components in Figs. 1 and 4 are identified with like character references. As shown in Fig. 5, de battery 19 provides a positive operating voltage $V_{x1}$. In this standby condition, the circuit draws a supply current of less than 1 microampere. The transmitter is switched on by closure of switch 18A which sets a flip/flop FF1 which activates a transistor T1 by way of a resistor 25 for providing operating voltage $V_2$. Activation of transistor T1 energizes a voltage converter VC which provides a negative operating voltage V-. The positive operating voltage $V_2$ is monitored by a low battery detector 47. This detector includes a comparator 50 which may take the form of an integrated circuit provided by Intersil and identified by their model No. ICL7665CP. Comparator 50 compares the voltage as supplied by the voltage dividing network made up of resistors R31, R32 and R33. If the voltage is too low, it triggers a flip/flop FF2 which, in turn, triggers a following flip/flop FF3 to note the low battery condition. The output of flip/flop FF3 turns on a transistor T6 by way of a resistor R42 to activate a light-emitting diode D12 to provide a yellow light to the operator, indicative of a low battery condition. In addition, the output of flip/flop FF3 turns on a transistor T5 by way of a diode D9 and resistor R38. With this transistor turned on, it activates a buzzer timer BT which, in turn, energizes a buzzer B1 for a predetermined time period to provide the operator with an audible indication of a low battery condition.

In addition to energizing the low battery yellow light and the audible buzzer, the output of flip/flop FF3 also resets flip/flop FF1 by way of diode D8 thereby turning transistor T1 off and de-energizing the voltage converter VC. This removes the operating voltage $V_2$ and V- and the circuit reverts to its standby condition.

After the switch 18A has been closed and providing a low battery condition is not detected, the pulse generator PG is activated to switch the transmitter on for one millisecond every 700 milliseconds. This is the pulse mode of operation which will be in effect until the transmitter has been correctly positioned relative to the implant. As previously indicated, the energy transmitted during this period is not sufficient to cause capacitor 14 (Fig. 3) in the receiver to be charged to a level sufficient to cause a pump action. The pulse generator operates in this mode until the position detector 25 has detected that the transmitter is correctly positioned.

The pulse generator is activated by turning on the operating voltage $V_2$. The generator includes a pulse timer PT which may take the form of an Inersil integrated circuit Model ICM7555. The output of the pulse timer PT is supplied through NAND gates 52 and 54. The output of NAND gate 54 serves as a clock for a flip/flop FF4, to be described hereinafter. The output of NAND gate 52 is employed for controlling the operation of the antenna transistor switch T2 by way of resistor R4.

The transmitter section 23 includes a crystal oscillator 0 followed by a switched transistor stage connected to the antenna 4. The oscillator 0, as shown in Fig. 5, is a conventional oscillator circuit operating at a frequency of 2 MHz and its output is supplied through a NOR gate 56 and this is supplied as one input to a NOR gate 58. The output of the NAND gate 52 of the pulse generator PG is inverted by a NOR gate 60 and its output is applied as the second input to NOR gate 58. During the pulse mode of operation, the output pulses from the pulse generator PG control the turn on of transistor T2 so that the oscillator pulses will be passed by NOR gate 58 to turn on the transistor. With the transistor turned on, the antenna 4 is energized when current flows through inductors L1, L2 and thence through the transistor. The antenna stage is comprised of inductor L2 and capacitor C5 and C6 and is tuned to 2 MHz. Whenever the transistor T2 is switched on, the transmitter will radiate a sinewave at a frequency of 2 MHz.

The high frequency carrier transmitted by antenna 4 is detected by a normal high frequency detector 24 which includes diode D1, capacitors C7 and C8 and resistor R5. The negative part of the envelope of the high frequency carrier is employed during the pulse mode of operation for purposes of comparing the signal to a preset level by means of the position detector 25. As shown in Fig. 5, the position detector receives an output from the high frequency detector 24 as one input to a comparator 62 for comparison to a preset level as set by a potentiometer P1. The comparator 62 includes a PMI integrated circuit Model CMP02CP. So long as the transmitting antenna 4 is not in its correct position relative to the receiving antenna 12, there is no loading on the transmitter and, hence, the output of the comparator 62 provides negative going pulses which do not charge the capacitor C17. As seen in the drawings, this capacitor is connected to the "D" input of flip/flop FF4 which is clocked by the pulse generator PG. When the transmitting antenna is properly positioned relative to the receiving antenna, the transmitter load will increase, causing the output of comparator 62 to stay high. This allows flip/flop FF4 to change states to switch the transistor stage through the NAND gate 52 to a continuous mode. Now, the transmitter will continue to transmit the 2 MHz signal on a continuous basis. In addition to switching the transmitter to its continuous mode, the flip/flop FF4 turns on transistor T3 by way of diode D4 and resistor R19 to thereby energy the "ready" indicator, taking the form of a light-emitting diode D6 so as to provide a continuous green signal to the operator. Prior to this, the transistor T3 was intermittently turned on to provide a flashing green signal with diode D6 by means of a circuit including the time-out counter TO by way of diode D5. In this flashing mode, the timeout counter TO receives pulses from the pulse generator PG.

When the flip/flop FF4 changed state, it also enabled a NAND gate 64 to pass pulses from the feedback detector 26 to a pulse counter PC by way of a NOR gate 66. The pulse counter may include a Philips integrated circuit Model HEF4024. The feedback detector 26 is connected to the output of the high frequency detector 24 by means of a differentiating circuit comprised of resistor R6, capacitor C9 and resistors R7 and R8, as shown in Fig. 5. As discussed previously, each pump action causes a sudden change in the transmitter load and this appears as a short step in the detected high frequency signal. This signal is differentiated by the differentiator circuit and appears as a short spike across resistor R7. This causes a positive going output signal on the output of a comparator 68 located in the feedback detector 26. This comparator may include a PMI integrated circuit Model CMP02CP. Each pump action produces one positive pulse at the output of the feedback detector 26 and this is passed by the enabled NAND gate 64 with the output thereof being inverted by NOR gate 66 and supplied as an input pulse to the pulse counter PC. The counter PC is preset to count a specific number of pulses corresponding with the number of pump actions and, hence, the dosage to be given to the patient in response to actuating the switch 18A. When that preselected number of pulses has been counted, the pulse counter PC triggers flip/flop FF5 which, in turn, provides an output to reset the power switch flip/flop FF1 (switch 18). This turns off the voltage converter VC, removing voltage sources $V_2$ and V- from the circuit. Additionally, with the voltage $V_2$ being removed from the circuit, NAND gate 70 resets flip/flop FF4 to remove the "READY" indication by deenergizing transistor T3. In addition to resetting flip/flop FF1, this also causes the flip/flop FF5 to momentarily turn on transistor T5 to energize the buzzer timer BT to energize buzzer B1 for a short period of time until the voltage $V_2$ is removed and flip/flop FF5 is reset.

The time-out circuit TO takes the form of a counter, as illustrated in Fig. 5, and this counter is activated upon power up to commence counting the pulses from the pulse generator PG and to monitor the output of the feedback detector 26 by way of the output of NOR gate 66. The counter includes a Philips integrated circuit Model HEF4024. The time-out counter counts the pulses from the pulse generator PG to define a given time interval. If no pulses are supplied from the output of NOR gate 66 representing a pump action within this time limit, the time-out counter TO resets the power switch 18 and, in addition, turns on a failure indicator. As seen in Fig. 5, power switch 18 also includes a second flip/flop FF6 which is reset by the time-out counter TO to turn on transistor T4 permitting the failure light-emitting diode D7 to be energized to provide a failure indication to the operator. The operator is also alerted by an audible signal, since the flip/flop FF6 additionally turns on transistor T5 to energize the buzzer timer BT which then turns on the audible buzzer B1 for a time period on the order of five seconds.

In the description given thus far with reference to Fig. 5, it will be noted that various of the NAND gates and NOR gates are shown as somewhat discrete components when they may be implemented as portions of an integrated circuit. For example, NOR gates 56, 58, 60 and 66 each comprise one-fourth of an integrated circuit, such as Philips Model No. HEF4001 which is sometimes known in the art as a quad 2 input NOR gate. Similarly, NAND gates 52, 54, 64 and 70 may each take the form of one-fourth of an integrated circuit such as that provided by Philips Model No. HEF4093 and which is sometimes referred to as a quad 2 input NAND gate. Also, flip/flops FF1 and FF6 may each take the form of one-half of an integrated circuit provided by Philips as Model No. HEF4013. Flip/flops FF2 and FF3 may also each take the form of one-half of an integrated circuit such as that of Philips Model No. HEF4013. Likewise, flip/flops FF4 and FF5 may each take the form of one-half of an integrated circuit such as that of Philips Model No. HEF4013. The various integrated circuits employed in Fig. 5 are illustrated herein with the conventional pin number locations in the interest of presenting a complete disclosure thereof.

Reference is now made to Fig. 6 which illustrates a microprocessor based transmitter system 100 in accordance with the present invention. Like components in Figs. 4, 5 and 6 are identified with like character references. As in the embodiments of Figs. 4 and 5, the operation commences upon closure of switch 18A which activates the power switch 18 from the battery 19 to provide circuit power. A low battery detector 47 operates in the same fashion as the detector 47 illustrated in Figs. 4 and 5 and provides a low battery indication to the microprocessor 20. The microprocessor is programmed so that in response to a low battery condition, it will deactivate the power switch 18 and activate a suitable acoustic indicator 27 and an optical indicator 28 in the same manner as activating the yellow light-emitting diode D12 and the buzzer B1 in Fig. 5. As will be observed, the microprocessor version of Fig. 6 performs the same functions as that in the circuitry of Fig. 5, but is more flexible and its specific operating programs may be downloaded into its memory as from a personal computer by way of a conventional RS 232 interface 48. The programmed microprocessor performs the functions of the flip/flops, counters and timing circuits of Fig. 5, including that of the pulse generator PG, the pulse counter PC, time-out circuit TO and the buzzer timer BT.

As in the case of the embodiment of Fig. 5, the circuitry of Fig. 6 includes a pulse mode operation during which the transmitter 23 intermittently transmits RF signals to the implanted receiver. The RF energy is not sufficient to charge the capacitor 14 in the receiver (Fig. 3) by an amount to cause a pump action. During this pulse mode, the transmitter is being positioned relative to the receiver to determine the optimum position. As the transmitting antenna is moved about the area in question, a position detector 25 responds to the output of the high frequency detector 24, constructed as illustrated in Fig. 5. When the loading of the transmitting antenna increases sufficiently, the position detector 25 will convey this information to the microprocessor. The microprocessor is programmed to, in turn, activate the transmitter to transmit at its continuous mode of operation and activate the acoustic indicator 27 and the optical indicator 28 in the same manner as described hereinbefore with respect to Fig. 5 wherein the acoustic indicator buzzer B1 was activated and the green ready diode D6 was turned on continuously to indicate a ready condition.

The feedback indicator 26 operates in the same manner as illustrated in Fig. 5 and operates to detect each pump action. For each detected pump action, the feedback detector 26 supplies a pulse to the microprocessor 20 which is programmed to count the number of pulses as in the manner described hereinbefore with respect to the pulse counter PC of Fig. 5. When the selected number of pump actions for the patient have been counted, the microprocessor will de-energize the power switch while at the same time activate the acoustic indicator 27 in the same manner as the activitization of the buzzer B1 with reference to Fig. 5. The microprocessor 20 is also programmed to perform the function of the time-out circuit TO of Fig. 5 to determine whether a pump action has been detected within a given period time subsequent to the closure of switch 18A. If this does not occur, then the microprocessor deactivates the power switch to remove circuit power and at the same activates the optical indicator 28 in the same manner as activating th failure red light-emitting diode D7 in Fig. 5. Simultaneously with indicating this failure condition, the microprocessor activates the acoustic indicator 27, as in activating the buzzer B1 in Fig. 5, to indicate to the operator that a failure condition has taken place.

From the foregoing it is seen, then, that the microprocessor version of Fig. 6 performs all of the functions of the circuitry illustrated in Fig. 5. In addition, the RS232 interface 48 permits the microprocessor to be coupled with a personal computer so that an operator may communicate with the processor's memory to vary the permissible number of doses per time limit and the limits within which the patient may take action.

In addition to the above, it is emphasized that the infusion device according to the invention is particularly suitable for pain treatment purposes by bolus delivering mode of fluid analgesics such as morphine, etc. and administered by the patient himself.

With respect to pain treatment in general, it is observed that pain to a patient is a very individual experience which may vary greatly from day to day or even still shorter periods dependent upon mental resistance, momentarily psychological mood, biorhytehm, etc. Accordingly, the infusion system used should be of sufficient flexibility to enable the drug delivery to vary between broad limits. On the other hand, while the delivery of an overdose of an analgesic such as morphine is perilous, an infusion system for local pain treatment should include the possiblity to prevent the patient to administer himself an overdose of the analgesic such as morphine.

The infusion system described and illustrated herein enables the control unit to be programmed in accordance with the medical attendant's wishes for each individual patient. Detached from the personal computer, the control unit can operate as an independent system. The pumping member operates according to a program read in memory. The patient receives programmed protection from overdoses. The pump functions may be recorded to be read out later via a computer. Defects, such as a clogged catheter or defects in the pump function may be indicated. Discrepancies between the calculated supply of the liquid material to be pumped may be audibly and/or visibly reported to the patient. Also, whether the pump functions properly or whether the transmitter battery is to be replaced or whether the reservoir is to be refilled may be noted.

In addition to the above-presented description of the infusion device, it is observed that when the antenna of the external part of the infusion system is applied to the patient's skin in optimum fixed relationship with respect to the implanted housing, the infusion system is in a condition for basal delivery mode by means of continuously delivering a series of constant bolusses. Such an embodiment of the infusion system is used in cases of chemotherapy by infusing medicaments such as adriamycine or 5 FU, etc. The infusion system according to the invention via its microprocessor version offers the possibility to be accordingly programmed as well as that it offers the possibility of a programmed liquid drug delivering mode being a combination of a basal delivery mode and a bolus delivery mode as is often required in case of administering insulin. To repeat once again, all the above-mentioned possibilities of delivering modes may be met by the infusion system according to the invention.

Although the invention has been described in conjunction with preferred embodiments, it is to be appreciated that various modifications and arrangements may be made without departing from the spirit of the invention as defined by the appended claims.

## Claims

1. An infusion system for infusion of fluids into a living body comprising:

an implantable housing to be implanted within a said body and containing a reservoir of said fluid and actuatable pump means for, when actuated, pumping a quantity of said fluid for delivery into said body; and

normally electrically passive pump actuation control means mounted within said housing for actuating said pump means and including receiving antenna means for receiving an RF signal through said body from an external transmitter, means for converting said RF signal into a DC signal, a capacitor and means responsive to said DC signal for charging said capacitor, voltage comparison means for comparing the charge level of said capacitor with a reference level and providing a trigger signal when said levels attain a predetermined relationship a capacitor discharge circuit including a first actuatable switch means, actuatable by a said trigger signal, and said actuatable pump means for discharging said capacitor and actuating said actuatable pump means to thereby pump a said quantity of said fluid for delivery into said body only in response to said receiving antenna means having received a said RF signal.

2. A system as set forth in claim 1 including second switch means for short-circuiting said receiving antenna means in response to actuation of said pump means to thereby affect a loading on a said external transmitter.

3. A system as set forth in claim 2 including mechanical actuation means responsive to a said pump action for connecting said switch means to short-circuit said receiving antenna means.

4. A system as set forth in claim 1 wherein said means for converting said RF signal into a DC signal includes voltage doubling circuit means.

5. A system as set forth in claim 1 in combination with an external transmitter having means for transmitting a said Rf signal and adapted to be moved in close proximity to a said body containing a said implantable housing.

6. A system as set forth in claim 5 wherein said transmitter includes a transmitting antenna means and position detector means for ascertaining an optimal position of said transmitting antenna means relative to said implantable housing as a function of the electrical load on said transmitting antenna.

7. A system as set forth in claim 6 wherein said position detector means includes means responsive to said load for comparing the said load with a reference and providing a ready output when said load is greater than said reference indicative of a close proximity of said transmitting and receiving antenna means.

8. A system as set forth in claim 7 wherein said transmitter includes power on switch means for, when actuated, turning on said transmitter for transmitting said RF signal, and transmitter control means responsive to turn on of said transmitter for in initially causing said transmitter to intermittently transmit said RF signal at a rate insufficient to charge said capacitor in said implantable housing to cause a said pump action and said transmitter control means being responsive to a said ready output for causing said transmitter to continuously transmit said RF signal.

9. A system as set forth in claim 8, including ready indicator means responsive to said ready output signal for providing a ready indication.

10. A system as set forth in claim 9 wherein said ready indicator means includes visually ready display means.

11. A system as set forth in claim 9 wherein said ready indicator means includes audible ready means for emitting an audible signal.

12. A system as set forth in claim 2 in combination with an external transmitter having transmitting antenna means for transmitting a said RF signal.

13. A system as set forth in claim 12 wherein said transmitter has pump action verification means for verifying that a said pump action has taken place.

14. A system as set forth in claim 13 wherein said verification means includes means responsive to the loading of said transmitting antenna for providing a verification output pulse indicative that a said pump action has taken place.

15. A system as set forth in claim 14 including verification counting means for counting the number of verification output pulses and providing a transmitter turn off signal upon counting a preselected number of said verification output pulses and means responsive to said turn off signal for turning off said transmitter.

16. A system as set forth in claim 15 including programmed computer means including said verification output pulse counting means for providing said turn off signal.

17. A system as set forth in claim 16 wherein said computer means includes memory means programmable to store said preselected number.

18. A system as set forth in claim 17 including RS232 interface means for interfacing said computer means and memory means with an external computer.

19. A system as set forth in claim 14 including time-out counting means for providing a failure output signal if a said verification output pulse is not provided within a preselected time period and means responsive to said failure output signal for turning off said transmitter and for providing a failure indication.

20. A system as set forth in claim 19 including a programmed computer means including said time-out counting means for providing said failure signal.

EP 0 300 552 A1

**FIG.1**

**FIG.2**

RECEIVING
ANTENNA

ELECTRONIC
SWITCH

DETECTOR

CAPACITOR

VOLTAGE
COMPARATOR

ELECTRONIC
SWITCH

PUMP

FIG.3

FIG.4

FIG.5

EP 0 300 552 A1

FIG.6

EP 0 300 552 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | EP-A-0 048 423 (SIEMENS AG) <br> * Figure 4 * | 1,4,12-18 | A 61 M 5/14 |
| Y | US-A-4 058 857 (T.M. RUNGE et al.) <br> * Column 2, lines 14-23; figure 2 * | 1,4,12-18 | |
| A,D | US-A-4 373 527 (FISCHELL) <br> * Abstract * | 1 | |
| A,D | US-A-4 360 019 (PORTNER et al.) <br> * Figure 5 * | 1 | |

TECHNICAL FIELDS
SEARCHED (Int. Cl.4)

A 61 M
H 02 J

The present search report has been drawn up for all claims

| Place of search | Date of completion of the search | Examiner |
|---|---|---|
| THE HAGUE | 30-09-1988 | EHRSAM F.J.A. |

EPO FORM 1503 03.82 (P0401)